# EUROPEAN PATENT SPECIFICATION

(11) **EP 1 362 603 B1**
(45) Date of publication and mention of the grant of the patent: **01.03.2006**
(21) Application number: 03010570.4
(22) Date of filing: 12.05.2003
(51) Int. Cl.: A61L 31/16, A61L 31/14, A61L 31/10, A61F 2/06

(54) **Coated stent for release of active agents**
Beschichteter Stent für die Freisetzung aktiver Substanzen
Stent revêtu pour la libération d'un principe actif

(30) Priority: 14.05.2002 JP 2002138735
(43) Date of publication of application: 19.11.2003
(73) Proprietor: TERUMO KABUSHIKI KAISHA, Tokyo 151-0072 (JP)
(72) Inventor: Togawa, Hideyuki, Ashigarakami-gun, Kanagawa 259-0151 (JP); Nagura, Hiroaki, Ashigarakami-gun, Kanagawa 259-0151 (JP)
(74) Representative: Casalonga, Axel

(56) References cited:
- EP-A- 0 734 721
- EP-A- 1 273 314
- WO-A-00/45744
- US-A- 5 447 724
- US-A- 5 624 411
- THE MERRIAM-WEBSTER DICTIONARY, HOME AND OFFICE EDITION , 1995 * page 404 *

## Description

### BACKGROUND OF THE INVENTION

### 1. Field of the Invention

The present invention relates to a stent to be used to treat a stenotic or occluded lesion that has occurred in such ducts of a living body as blood vessel, bile duct, trachea, esophagus, and urethra.

### 2. Description of the Related Art

One example of such treatments is the angioplasty which is applied to ischemic heart diseases as explained in the following.

Widespread westernized eating habits in Japan are responsible for the recent rapid increase in patients suffering from ischemic heart diseases, such as angina pectoris and cardiac infarction. One way to treat such coronary lesions is percutaneous transluminal coronary angioplasty (PTCA), which is gaining general acceptance rapidly. Because of its technical development, it is now applied to more cases than before. PTCA was originally applied to a circumscribed lesion (with short lesion length) or a single vessel lesion (with the stenosis only at one vessel). Its application has been extended to a lesion which is distal, eccentric, and calcified, or a multivessel lesion (with the stenosis at two or more vessels). PTCA is a procedure which consists of making a small incision in an artery of a patient's arm or leg, indwelling an introducer sheath in the incision, inserting a long tube called guide catheter into the blood vessel through the sheath, with a guide wire preceding, after the tube reaches the entrance of the coronary artery, withdrawing the guide wire, passing another guide wire and a balloon catheter through the guide catheter, advancing the balloon catheter under the X-ray radiography, with the guide wire preceding, to the lesion, namely the stenotic or occluded lesion in the coronary artery, and inflating the balloon once or more times at a prescribed pressure for 30-60 seconds. The result of this procedure is that the blood vessel at the lesion expands and remains open, thereby allowing more blood to flow in the blood vessel. The disadvantage of this procedure is that there is a possibility of the catheter damaging the vascular wall. In fact, it has been reported that restenosis occurs in a ratio of 30-40% by the growth of the vascular inner membrane which heals the damaged vascular wall.

Although no method for preventing restenosis has been firmly established yet, there is a new method which is under study and somewhat successful so far. It employs such devices as stent and atherectomy catheter. The term "stent" used herein denotes a medical device in hollow cylindrical shape, usually made of a metallic or polymeric material. A stent is used to cure various diseases resulting from stenosis or occlusion in blood vessels or other ducts. When in use, it expands the lesion, namely the stenotic or occluded part and is implanted there so as to keep open the blood vessel or duct. A variety of stents have been proposed so far. For example, one is a hollow cylindrical body having pores formed in the side wall thereof which is made of a metallic or polymeric material, and another is a cylindrical body which is woven from metal wires or polymer fibers. The object of the implanted stent is to prevent or reduce the possibility of restenosis occurring after PTCA has been performed. However, it has turned out that any stent used alone cannot effectively prevent restenosis.

Nowadays, various attempts are being made to reduce the possibility of restenosis by using an improved stent loaded with a biologically/physiologically active substance (such as anticancer drug) which releases itself over a long period of time at the implanted lesion in a duct. An example of the stent is disclosed in US 5,464,650 A and US 5,837,008 A; it is coated with a mixture composed of a therapeutical substance and a polymer. Another example is disclosed in JP 9-056807 A; it is covered sequentially with a drug layer and a biodegradable polymer layer.

However, the stent proposed in US 5,464,650 A and US 5,837,008 A has the disadvantage that the therapeutical substance (biologically/physiologically active substance) incorporated in the polymer is subject to decomposition or deterioration by chemical reactions between them. In other words, the stent poses a problem associated with instability of biologically/physiologically active substance. This problem is explained below with reference to a case (as an example) in which the polymer is polylactic acid. Because of its tendency toward decomposition in a living body, polylactic acid permits the biologically/physiologically active substance to release itself efficiently in a living body. On the other hand, because of its ability to liberate an acid upon decomposition, polylactic acid deteriorates the biologically/physiologically active substance which might have a weak acid resistance. Moreover, any readily biodegradable polymer is not desirable because it permits the biologically/physiologically active substance to release itself in a short time (several days after implanting) and hence disables the stent from preventing restenosis. In other words, the stent proposed in US 5, 464, 650 A and US 5, 837, 008 A, which is so designed as to protect the biologically/physiologically active substance from decomposition and deterioration and to permit the biologically/physiologically active substance to release itself over a long period of time (about two months after implanting), has the disadvantage of limiting the range of selection of the biologically/physiologically active substance that can be combined with the polymer.

By contrast, the stent proposed in JP 9-56807 A, which has a layer of drug (biologically/physiologically active substance) and a layer of biodegradable polymer formed separately, is satisfactory in view of the fact that the biologically/physiologically active substance is exempt from decomposition and deterioration by the polymer. Nevertheless, it was found that the stent causes 30% of its biologically/physiologically active substance to release itself within one day after immersion in serum of a bovine fetus. Usually, PTCA or stent implanting causes a local damage (such as peeling of endothelial cells or damage of elastic laminae arteries) to the blood vessel in question. Presumably, it takes a relatively long period (about two months after implanting of a stent) for the living body to heal such a damage. To be more specific, restenosis is considered to be ascribed to both inflammation seen as adhesion and infiltration of monocytes, which occurs within 1-3 days after PTCA or stent implanting, and thickening of inner membrane with smooth muscle cells whose growth reaches a peak on the 45th day or so. Since the growth of smooth muscle cells is the major cause of restenosis, it seems most effective to prevent the growth of smooth muscle cells within a period from the 30th day (when the growth of smooth muscle cells are noticed in inner membrane by pathological diagnosis) to the 45th day (when the growth rate reaches a peak). (ref. International J. of Cardiology 1996; 53.71:, Heart 2003; 89: 133.) In other words, it is most desirable for a stent to be able to release the biologically/physiologically active substance variably depending on the period after implanting. That is, there should be a maximum rate of release in the first period within 10 days (for prevention of inflammation) and in the second period within 30-60 days (for prevention of growth of smooth muscle cells), and there should be a uniform rate of release (enough for drug effect) throughout the rest of the period. Consequently, the stent disclosed in JP 9-56807 A mentioned above, which releases 30% of its biologically/physiologically active substance within one day after immersion inserumof a bovine fetus, has the disadvantage of being unable to release its biologically/physiologically active substance sufficiently within the period for prevention of the growth of smooth muscle cells, although it releases its biologically/physiologically active substance sufficiently in the period for prevention of inflammation. For a stent to release its biologically/physiologically active substance within the period for prevention of the growth of smooth muscle cells, it should be made of a biodegradable polymer which is comparatively slow in decomposition in a living body. Unfortunately, such a stent cannot prevent inflammation because the sustained release of its biologically/physiologically active substance is limited by the rate of decomposition of the biodegradable polymer in the early period.

### SUMMARY OF THE INVENTION

It is an object of the present invention to provide a stent which is composed of a stent main body and a biologically/physiologically active substance stably supported thereon without the possibility of decomposition and degradation, the biologically/physiologically active substance releasing itself at a maximum rate within two periods of at least 10 days and 30-60 days after implanting at a lesion.

In carrying out the invention and according to an aspect thereof, there is provided a stent for implanting in a duct of a living body including: a stent main body; a biologically/physiologically active substance layer formed from at least one kind of biologically/physiologically active substance and provided on the surface of the stent main body; a polymer layer of biodegradable polymer completely covering the biologically/physiologically active substance layer; and a water-soluble substance contained in the polymer layer in dispersed state, the water-soluble substance being to be eluted in the living body; wherein pores are formed in the polymer layer with the elution of the water-soluble substance; the pores control the initial release of the biologically/physiologically active substance; and the decomposition of the biodegradable polymer controls the secondary release of the biologically/physiologically active substance.

With this configuration, the biologically/physiologically active substance is not exposed to outside air, nor is it mixed with the polymer. (It is in contact with the polymer only at their interface.) Therefore, the biologically/physiologically active substance remains stable, without decomposition or deterioration, on the stent main body. If the polymer (for the polymer layer) and the water-soluble substance have an adequate composition and molecular weight, the resulting stent will permit the biologically/physiologically active substance to release itself at a maximum rate in the first period within 10 days (for prevention of inflammation) and in the second period within 30-60 days (for prevention of growth of smooth muscle cells). In addition, the polymer layer is completely decomposed in and absorbed by a living body, with no foreign matter left behind.

Preferably, the stent main body is formed from a metallic material with high strength. In this case, the resulting stent ensures implanting at a lesion.

Preferably, the stent main body is formed from a polymeric material with good flexibility. In this case, the resulting stent ensures easy delivery to a lesion.

Preferably, the biologically/physiologically active substance layer is composed solely of a biologically/physiologically active substance. Inthiscase, the layer can be formed in a simple manner.

Preferably, the biologically/physiologically active substance layer is formed from a mixture of a biologically/physiologically active substance and a low-molecular weight water-soluble material. In this case, the layer has improved adhesion to the stent main body.

Preferably, the biologically/physiologically active substance is at least one member selected from the group consisting of carcinostatic, immunosuppressive, antibiotic, antirheumatic, antithrombotic, antihyperlipidemic, ACE inhibitor, calcium antagonist, integrin inhibitor, antiallergic, antioxidant, GPIIb/IIIa antagonist, retinoid, flavonoid, carotenoid, lipid improving agent, DNA systnesis inhibitor, tyrosinekinase inhibitor, antiplatelet, vascular smooth muscle antiproliferative agent, antiinflammatory agent, living body-derived material, interferon, and NO production accelerator. In this case, the stent is effective in preventing restenosis.

Preferably, the biodegradable polymer is any of polylactic acid, polyglycolic acid, polyhydroxybutyric acid, and polycaprolactone, or a mixture of two or more thereof in which components are simply mixed together or covalently bonded together.

Preferably, the water-soluble substance is a water-soluble polymer.

Preferably, the water-soluble polymer is at least one member selected from the group consisting of water-soluble polyalkylene glycol, polyvinylpyrrolidone, polysaccharide, polyvinyl alcohol, polyacrylamide, and polyacrylate salt.

Preferably, the water-soluble substance is a water-soluble organic compound.

Preferably, the water-soluble organic compound is glycerin or propylene glycol.

The above and other objects, features and advantages of the present invention will become apparent from the following description and the appended claims, taken in conjunction with the accompanying drawings in which like parts or elements denoted by like reference symbols.

### BRIEF DESCRIPTION OF THE DRAWINGS

Fig. 1 is a front view showing one embodiment of the stent according to the present invention.
Fig. 2 is an enlarged transverse cross-sectional view taken along the line A-A in Fig. 1.
Fig. 3 is a partly enlarged vertical cross-sectional view taken along the line B-B in Fig. 2.
Fig. 4 is a partly enlarged vertical cross-sectional view illustrating the initial stage of elution of the water-soluble substance from the polymer layer of the stent shown in Fig. 3.
Fig. 5 is a partly enlarged vertical cross-sectional view illustrating the formation of pores in the polymer layer of the stent shown in Fig. 3.
Fig. 6 is a graph in which the amount (in percentage) of simvastatin released in human plasma is plotted against the number of elapsed days.

### DESCRIPTION OF ILLUSTRATIVE EMBODIMENTS

The invention will be described in more detail with reference toillustrative embodiments in conjunction with the accompanying drawings.

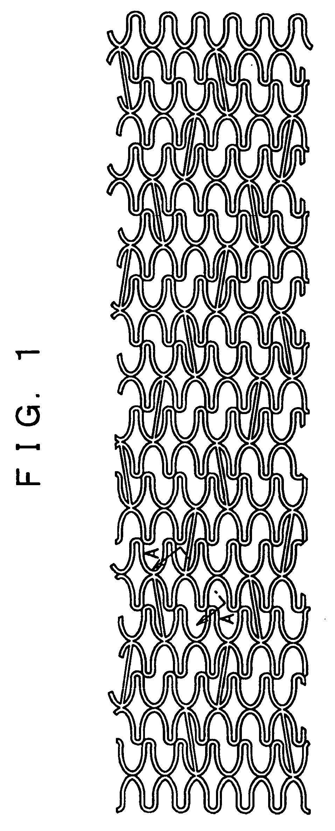

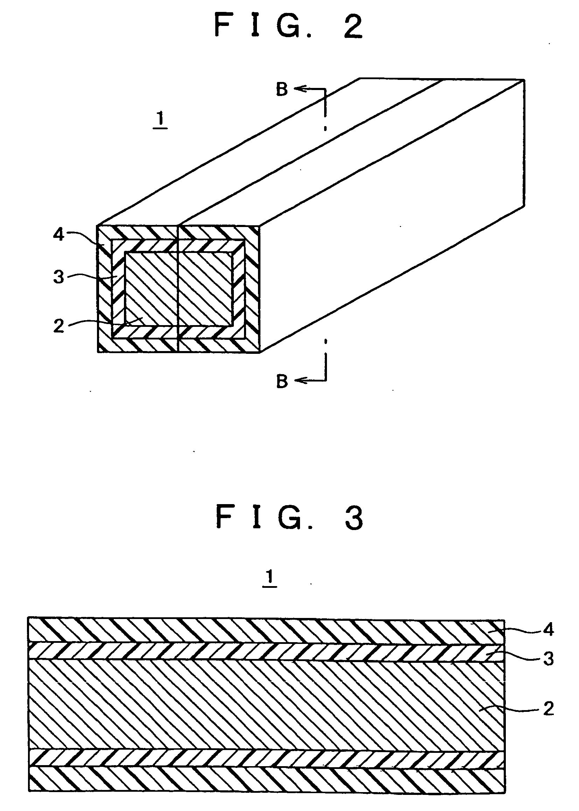

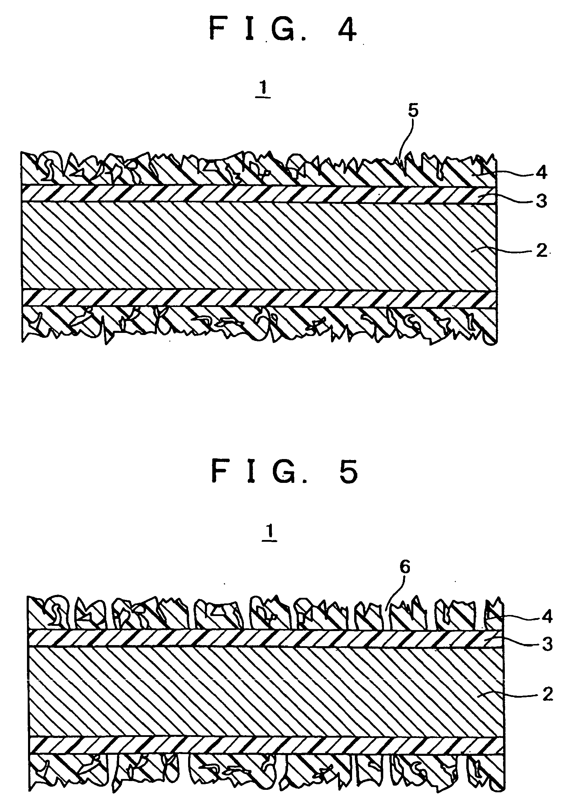

Fig. 1 is a front view showing one embodiment of the stent according to the present invention. Fig. 2 is an enlarged transverse cross-sectional view taken along the line A-A in Fig. 1. Fig. 3 is a partly enlarged vertical cross-sectional view taken along the line B-B in Fig. 2. Fig. 4 is a partly enlarged vertical cross-sectional view illustrating the initial stage of elution of the water-soluble substance from the polymer layer of the stent shown in Fig. 3. Fig. 5 is a partly enlarged vertical cross-sectional view illustrating the formation of pores in the polymer layer of the stent shown in Fig. 3.

According to the present invention, the stent 1 shown in Figs. 2 and 3 is made up of a stent main body 2, a biologically/physiologically active substance layer 3, and a polymer layer 4. The biologically/physiologically active substance layer 3 is formed from at least one kind of biologically/physiologically active substance. The biologically/physiologically active substance layer 3 is completely covered with the polymer layer 4 formed from a biodegradable polymer. The polymer layer 4 also contains a water-soluble substance (not shown) dispersed therein which elutes in a living body. The stent 1 produces its effect as follows after it has been implanted at a lesion in a living body. First, the water-soluble substance begins to elute, forming pores (passages) 6 in the polymer layer. These pores 6 connect the biologically/physiologically active substance layer 3 to the outer surface of the polymer layer 4, so that they permit the biologically/physiologically active substance to release itself initially within a period of 10 days after implanting. How many and large pores 6 are formed depends on the amount of the water-soluble substance. Then, the polymer layer 4 formed from a biodegradable polymer decomposes with the lapse of time, thereby permitting the biologically/physiologically active substance to release itself secondarily within a period of 30-60 days after implanting.

The stent 1 is made up of several components which are explained in more detail in the following.

The stent main body 2 is not specifically restricted in material, shape, and size so long as it can be implanted at a lesion in a duct of a living body, such as blood vessel, bile duct, trachea, esophagus, and urethra. The implanting of the stent 1 may be accomplished by balloon expansion as mentioned above. Alternatively, it may be accomplished by self expansion if the stent main body 2 is made of an elastic material.

The stent main body 2 may be formed from a material such as a metallic material, a polymeric material, a carbon fiber or ceramics, which is selected according to the lesion to which the stent 1 is applied. Because of its high strength, a metallic material is desirable for the stent 1 which needs secure implanting. Because of its good flexibility, a polymeric material is desirable for the stent 1 which needs easy delivery to a lesion.

Examples of the metallic material include stainless steel, Ni-Ti alloy, tantalum, titanium, gold, platinum, inconel, iridium, tungsten, and cobalt alloy. Of stainless steel, SUS316L is most adequate because of its good corrosion resistance.

An ordinary metallic material gives a stent main body 2 which is usually expandable by a balloon. A metallic material, such as Ni-Ti alloy, which has pseudoelasticity, gives a stent main body 2 which expands by itself with its elastic force when it is implanted in its compressed form at a lesion and then released from compressive force after implanting. Pseudoelasticity is the property that a metallic material greatly changes in strain while stress is kept constant or a metallic material gradually changes in strain with an increasing stress.

Examples of the polymeric material include polytetrafluoroethylene, polyethylene, polypropylene, polyethylene terephthalate, cellulose acetate, cellulose nitrate (which is biocompatible), polylactic acid, polyglycolic acid, copolymer of lactic acid and glycolic acid, polycaprolactone, and polyhydroxybutyrate-valirate (which is biodegradable).

If formed from a biodegradable polymer, the resulting stent main body 2 disappears with the lapse of time after implanting on account of decomposition in a living body. This offers the advantage that a fresh stent can be implanted again at the same lesion.

The stent main body 2 is not specifically restricted in.shape so long as it has strength enough for the stent 1 to stably stay in a duct of a living body. Thus, it may be formed from metal wire or polymer fiber by braiding into a cylinder or it may be formed in a perforated tube from a metallic or polymeric material.

The stent main body 2 may be of either balloon-expanding type or self-expanding type. The size of the stent main body 2 should be properly selected according to the lesion to which the stent 1 is applied. For example, in the case of a stent for the coronary artery, the stent ma in body 2 should preferably measure 1.0-3.0 mm in outside diameter and 5-50 mm in length before expansion.

The stent main body 2 has its surface covered with a biologically/physiologically active substance layer 3 formed from at least one kind of biologically/physiologically active substance.

No specific restrictions are imposed on the method of forming the biologically/physiologically active substance layer 3 on the surface of the stent main body 2. Available methods include the one which comprises melting a biologically/physiologically active substance and coating the surface of the stent main body 2 with the resulting melt, the one which comprises dissolving a biologically/physiologically active substance in a solvent, dipping the stent main body 2 in the resulting solution, and removing the solvent by vaporization after pulling up, and the one which comprises spraying the stent main body 2 with the above-mentioned solution and removing the solvent by vaporization or the like.

Incidentally, adhesion of the biologically/physiologically active substance to the stent main body 2 may be ensured by incorporation with an adjuvant. Examples of adjuvants for water-soluble biologically/physiologically active substances include saccharides (monosaccharides, disccharides, and oligosaccharides), water-soluble vitamins, dextran, and hydroxyethylcellulose. Examples of adjuvants for fat-soluble biologically/physiologically active substances include low-molecular weight higher fatty acid (fish oil and vegetable oil) and fat-soluble vitamins (vitamin A and vitamin E).

The dipping method and spraying method mentioned above are simple and desirable if a solvent readily dissolves the biologically/physiologically active substance and readily wets the surface of the stent main body 2.

The biologically/physiologically active substance layer 3 should have an adequate thickness which does not produce any adverse effect on the performance of the stent main body 2, such as ease with which the stent is delivered to the lesion and non-irritant action on the blood vessel. The thickness should range from 1 to 100 µm, preferably from 10 to 50 µm, more preferably from 20 to 30 µm, so that the biologically/physiologically active substance fully produces its effect.

The biologically/physiologically active substance layer 3 may be formed from any biologically/physiologically active substance which is not specifically restricted so long as it produces the effect of preventing restenosis when the stent 1 is implanted at a lesion in a duct. Examples of the biologically/physiologically active substance include carcinostatic, immunosuppressive, antibiotic, antirheumatic, antithrombotic, antihyperlipidemic, ACE inhibitor, calcium antagonist, integrin inhibitor, antiallergic, antioxidant, GPIIb/IIIa antagonist, retinoid, flavonoid, carotenoid, lipid improving agent, DNA synthesis inhibitor, tyrosine kinase inhibitor, antiplatelet, vascular smooth muscle antiproliferative agent, antiinflammatory agent, living body-derived material, interferon, and NO production accelerator.

Exemplary carcinostatics include vincristine sulfate, vinblastine sulfate, vindesine sulfate, irinotecan hydrochloride, paclitaxel, docetaxel hydrate, methotrexate, and cyclophosphamid.

Exemplary immunosuppressives include sirolimus, tacrolimus hydrate, azathioprine, cyclosporin, mycophenolate mofetil, gusperimus hydrochloride, and mizoribine.

Exemplary antibiotics include mitomycin C, doxorubicin hydrochloride, actinomycin D, daunorubicin hydrochloride, idarubicin hydrochloride, pirarubicin hydrochloride, aclarubicin hydrochloride, epirubicin hydrochloride, peplomycin hydrochloride, and zinostatin stimalamer.

Exemplary antirheumatics include sodium aurothiomalate, penicillamine, and lobenzarit disodium.

Exemplary antithrombotics include heparin, ticlopidine hydrochloride, and hirudin.

Exemplary antihyperlipidemics include HMG-CoA reductase inhibitor and probucol. The former includes serivastatin sodium, atolvastatin, nisvastatin, pitavastatin, fluvastatin sodium, simvastatin, lovastatin, and pravastatin sodium.

Exemplary ACE inhibitors include quinapril hydrochloride, perindopril erbumine, trandolapril, cilazapril, temocapril hydrochloride, delapril hydrochloride, enarapril maleate, lisinopril, and captopril.

Exemplary calcium antagonist include nifedipine, nilvadipine, diltiazem hydrochloride, benipidine hydrochloride, and nisoldipine.

Exemplary antiallergics include tranilast.

Exemplary retinoids include all-trans retinoic acid.

Exemplary antioxidants include catechiness, anthocyanine, proanthocyanidine, lycopene, and β-carotene. Of the catechiness, the most preferred is epigallocatechin gallate.

Exemplary tyrosine kinase inhibitors include genistein, tyrphostin, and apstatin.

Exemplary antiinflammatory agents include steroids (such as dexamethasone and prednisolone) and aspirin.

Exemplary living body-derived materials include EGF (epidermal growth factor), VEGF (vascular endothelial growth factor), HGF (heptatocyte growth factor), PDGF (platelet derived growth factor), and BFGF (basic fibrolast growth factor).

The biologically/physiologically active substance constituting the biologically/physiologically active substance layer 3 should preferably contain at least one kind of the above-mentioned substances from the standpoint of certainly preventing restenosis. Their selection and combination should be made adequately according to the particular case.

The biologically/physiologically active substance layer 3 is completely covered with the polymer layer 4 of biodegradable polymer. In addition, the polymer layer 4 contains a water-soluble substance dispersed therein which is eluted in a living body.

No specific restrictions are imposed on the method of covering the biologically/physiologically active substance layer 3 with the polymer layer 4. Available methods include the one which consists of dissolving the polymer and water-soluble substance in a solvent and dipping the stent main body 2 (having the biologically/physiologically active substance layer 3 formed thereon) in the resulting solution, followed by drying, and the one which comprises spraying the stent main body 2 (having the biologically/physiologically active substance layer 3 formed thereon) with the above-mentioned solution, followed by drying.

The polymer constituting the polymer layer 4 is not specifically restricted so long as it is a biodegradable one which is highly stable in a living body. It includes, for example, polylactic acid, polyglycolic acid, polyhydroxybutyric acid, polycaprolactone, and a mixture of two or more thereof in which components are simply mixed together or covalently bonded together. Most desirable of these examples is polylactic acid or its copolymer with polyglycolic acid. An adequate one should be selected which ensures the sustained release of the biologically/physiologically active substance.

As with the biologically/physiologically active substance layer 3, the polymer layer 4 should have an adequate thickness which does not produce any adverse effect on the performance of the stent main body 2, such as ease with which the stent is delivered to the lesion and non-irritant action on the blood vessel. The thickness should range from 1 to 75 µm, preferably from 10 to 50 µm, more preferably from 20 to 30 µm.

With a thickness less than 1 µm, the polymer layer 4 would not completely cover the biologically/physiologically active substance layer 3, with a thickness more than 75 µm, the polymer layer 4 makes the stent 1 itself have an excessively large outside diameter which prevents the stent 1 from being delivered to the lesion smoothly.

No specific restrictions are imposed on the water-soluble substance so long as it dissolves easily in the humor (such as blood) without giving rise to any medically dangerous product. It may be either water-soluble polymer or water-soluble organic compound; however, it should preferably be one which dissolves in the solvent in which the biodegradable polymer constituting the polymer layer 4 dissolves. The water-soluble substance can be readily dispersed in the polymer layer 4 if it is dissolved together with the biodegradable polymer.

Examples of the water-soluble polymer include water-soluble polyalkylene glycol, polyvinyl pyrrolidone, polysaccharide, polyvinyl alcohol, polyacrylamide, and polyacrylate salt. Of these examples, water-soluble polyalkylene glycol is most desirable, because it is readily soluble in water and organic solvents and it is widely used as a pharmaceutical additive for its safety.

Examples of the water-soluble organic compound include glycerin and propylene glycol.

The concentration of the water-soluble substance should be 1-50 wt%, more preferably 5-30 wt%, of the weight of the biodegradable polymer constituting the polymer layer 4.

With a concentration more than 50 wt%, the water-soluble substance forms pores (passages) 6 in the polymer layer 4 in an early stage (several hours after implanting). Such pores connect the biologically/physiologically active substance layer 3 to the outer surface of the polymer layer 4, thereby making it difficult to control the initial release of the biologically/physiologically active substance. On the other hand, with a concentration less than 1 wt%, the water-soluble substance does not form pores 6 in the polymer layer 4. The absence of pores prevents the initial release of the biologically/physiologically active substance.

The biologically/physiologically active substance constituting the biologically/physiologically active substance layer 3 releases itself from the stent 1 in the following manner.

As soon as the stent 1 is implanted at a lesion, its outer surface, namely the outer surface of the polymer layer 4, comes into contact with the humor in the duct. As the result, the water-soluble substance existing near the outer surface of the polymer layer 4 begins to elute, forming irregularities 5 on the outer surface of the polymer layer 4 (Fig. 4). After that, the humor infiltrates into the polymer layer 4, thereby the water-soluble substance contained in the polymer layer 4 is eluted. Elution proceeds to such an extent that the pores (passages) 6 which connect the outer surface of the polymer layer 4 to the biologically/physiologically active substance layer 3 are formed (Fig. 5).

The pores 6 permit the humor in the duct to come into contact with the biologically/physiologically active substance layer 3. Thus the biologically/physiologically active substance dissolves in the humor and then releases itself from the stent 1 through the pores 6 formed in the polymer layer 4.

Subsequently, the polymer layer 4 gradually decomposes and the pores 6 grow larger. This results in an increase in the amount of the biologically/physiologically active substance which releases itself through the pores 6. The amount of the residual biologically/physiologically active substance in the stent 1 decreases with the lapse of time, and hence the amount of the biologically/physiologically active substance which releases itself from the stent 1 gradually decreases.

Eventually, the polymer layer 4 decomposes completely and the decomposition product is absorbed by the living body and the biologically/physiologically active substance releases itself completely in the living body.

As mentioned above, the stent 1 according to the present invention can be designed such that the biologically/physiologically active substance releases itself at a maximum rate in the first period within 10 days (for prevention of inflammation) and in the second period within 30-60 days (for prevention of growth of smooth muscle cells), by adequately selecting the composition and molecular weight of the biodegradable polymer (for the polymer layer 4) and the water-soluble substance.

In addition, the stent according to the present invention 1 does not pose the problem that the biologically/physiologically active substance is decomposed or deteriorated by the polymer because the biologically/physiologically active substance layer 3 is separate from the polymer layer 4. Therefore, the biologically/physiologically active substance remains stable in the stent main body 2 until it releases itself. There is no limitation on the combination of the polymer and the biologically/physiologically active substance.

### EXAMPLES

The invention will be described in more detail with reference to the following examples, which are not intended to restrict the scope thereof.

### Example 1

A stent sample was prepared in the following manner from a stent main body in cylindrical shape, 1.8 mm in outside diameter and 30 mm long (as shown in Fig. 1), which is made of SUS316L. This stent main body was sprayed with a 20 wt% solution of simvastatin (SV for short) (which is an antihyperlipidemic) dissolved in tetrahydrofuran (THF for short), by using a hand spray (HP-C made by Iwata). It was confirmed that the stent main body was coated with about 500 µg of SV after THF had been dried off. Thus there was formed a biologically/physiologically active substance layer, about 3 µm thick on average. The coated stent main body was sprayed with a 10 wt% solution of a 7:3 mixture of polylactic acid (M.W. = 50,000) (PLLA 50000 for short) and polyethylene glycol (M.W. =20,000, NacalaiTesque) (PEG20000 for short) dissolved in dichloromethane (DCM for short), by using the same hand spray as mentioned above. Spraying was followed by drying in a vacuum to completely evaporate dichloromethane. It was confirmed that the biologically/physiologically active substance layer was completely covered with the polymer layer. Incidentally, the amount of the polymer coating on the stent was 1 mg, and the thickness of the polymer layer was 50 µm on average.

The thus obtained stent sample was measured for the rate at which the biologically/physiologically active substance (SV) releases itself.

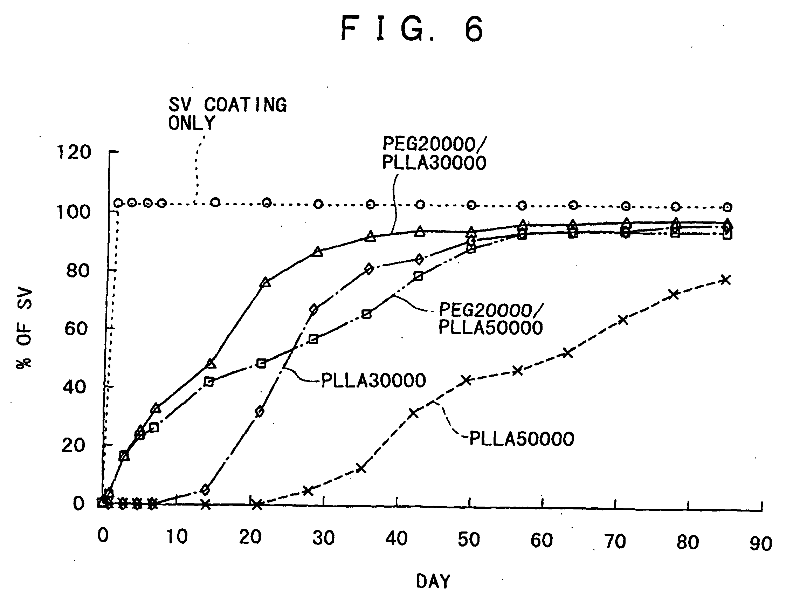

Measurement was carried out in the following manner. The stent sample is immersed in 4 ml of human plasma with stirring at 37°C. Sampling is carried out at prescribed time intervals. Each sample is analyzed by HPLC (made by Hitachi) to determine the amount of SV which has released itself into the human plasma. The result is shown in Fig. 6. Incidentally, the amount of SV in Fig. 6 is expressed in terms of the ratio (%) to the amount of SV applied to the stent.

As shown in Fig. 6, it is observed that the initial release of SV takes place within 10 days after immersion in human plasma and the secondary release of SV takes place within 30-60 days after immersion in human plasma. In other words, it is found that the polymer layer composed of PEG 20000 and PLLA 50000 permits SV to release itself at a maximum rate on the 10th day and 45th day. This results in an ideal SV release curve.

### Example 2

A stent sample was prepared in the following manner from a stent main body in cylindrical shape, 1.8 mm in outside diameter and 30 mm long (as shown in Fig. 1), which is made of SUS316L. This stent main body was sprayed with a 20 wt% solution of simvastatin (SV for short) (which is an antihyperlipidemic) dissolved in tetrahydrofuran (THF for short), by using a hand spray (HP-C made by Iwata). It was confirmed that the stent main body was coated with about 500 µg of SV after THF had been dried off. Thus there was formed a biologically/physiologically active substance layer, about 3 µm thick on average. The coated stent main body was sprayed with a 10 wt% solution of a 7:3 mixture of polylactic acid (M.W. =30,000) (PLLA 30000 for short) and polyethylene glycol (M.W. =20,000, NacalaiTesque) (PEG 20000 for short) dissolved in dichloromethane (DCM for short), by using the same hand spray as mentioned above. Spraying was followed by drying in a vacuum to completely evaporate dichloromethane. It was confirmed that the biologically/physiologically active substance layer was completely covered with the polymer layer. Incidentally, the amount of the polymer coating on the stent was 1 mg, and the thickness of the polymer layer was 50 µm on average.

The thus obtained stent sample was measured (in the same way as in Example 1) for the rate at which the biologically/physiologically active substance (SV) releases itself. The results are shown in Fig. 6.

As shown in Fig. 6, it is observed that the initial release of SV takes place within 10 days after immersion in human plasma and the secondary release of SV takes place within 30-60 days after immersion in human plasma.

### Comparative Example 1

A stent sample was prepared in the following manner from a stent main body in cylindrical shape, 1.8 mm in outside diameter and 30 mm long (as shown in Fig. 1), which is made of SUS316L. This stent main body was sprayed with a 20 wt% solution of simvastatin (SV for short) (which is an antihyperlipidemic) dissolved in tetrahydrofuran (THF for short), by using a hand spray (HP-C made by Iwata) . It was confirmed that the stent main body was coated with about 500 µg of SV after THF had been dried off. Thus there was formed a biologically/physiologically active substance layer, about 3 µm thick on average.

The thus obtained stent sample was measured (in the same way as in Example 1) for the rate at which the biologically/physiologically active substance (SV) releases itself. The results are shown in Fig. 6.

As shown in Fig. 6, it is observed that SV entirely releases itself in one day after immersion in human plasma.

### Comparative Example 2

A stent sample was prepared in the following manner from a stent main body in cylindrical shape, 1.8 mm in outside diameter and 30 mm long (as shown in Fig. 1), which is made of SUS316L. This stent main body was sprayed with a 20 wt% solution of simvastatin (SV for short) (which is an antihyperlipidemic) dissolved in tetrahydrofuran (THF for short), by using a hand spray (HP-C made by Iwata). It was confirmed that the stent main body was coated with about 500 µg of SV after THF had been dried off. Thus there was formed a biologically/physiologically active substance layer, about 3 µm thick on average. The coated stent main body was sprayed with a 10 wt% solution of polylactic acid (M.W. = 50,000) (PLLA 50000 for short) dissolved in dichloromethane (DCM for short), by using the same hand spray as mentioned above. Spraying was followed by drying in a vacuum to completely evaporate dichloromethane. It was confirmed that the biologically/physiologically active substance layer was completely covered with the polymer layer. Incidentally, the amount of the polymer coating on the stent was 1 mg, and the thickness of the polymer layer was 50 µm on average.

The thus obtained stent sample was measured (in the same way as in Example 1) for the rate at which the biologically/physiologically active substance (SV) releases itself. The results are shown in Fig. 6.

As shown in Fig. 6, it is observed that SV does not release itself in 20 days after immersion in human plasma. SV begins to release itself later due to decomposition of the polylactic acid but releases itself only 50% of its amount in 60 days after immersion in human plasma.

### Comparative Example 3

A stent sample was prepared in the following manner from a stent main body in cylindrical shape, 1.8 mm in outside diameter and 30 mm long (as shown in Fig. 1), which is made of SUS316L. This stent main body was sprayed with a 20 wt% solution of simvastatin (SV for short) (which is an antihyperlipidemic) dissolved in tetrahydrofuran (THF for short), by using a hand spray (HP-C made by Iwata). It was confirmed that the stent main body was coated with about 500 µg of SV after THF had been dried off. Thus there was formed a biologically/physiologically active substance layer, about 3 µm thick on average. The coated stent main body was sprayed with a 10 wt% solution of polylactic acid (M.W. = 30,000) (PLLA 30000 for short) dissolved in dichloromethane (DCM for short), by using the same hand spray as mentioned above. Spraying was followed by drying in a vacuum to completely evaporate dichloromethane. It was confirmed that the biologically/physiologically active substance layer was completely covered with the polymer layer. Incidentally, the amount of the polymer coating on the stent was 1 mg, and the thickness of the polymer layer was 50 µm on average.

The thus obtained stent sample was measured (in the same way as in Example 1) for the rate at which the biologically/physiologically active substance (SV) releases itself. The results are shown in Fig. 6.

As shown in Fig. 6, it is observed that SV releases itself very little in 10 days after immersion in human plasma.

It is concluded from the above-mentioned results that the initial release of SV is due to elution of polyethylene glycol and the secondary release of SV can be controlled by varying the molecular weight of the biodegradable polymer.

## Claims

1. A stent for implanting in a duct of a living body comprising:
1) a stent main body;
2) a biologically/physiologically active substance layer formed from at least one kind of biologically/physiologically active substance and provided on the surface of said stent main body; and
3) a polymer layer of biodegradable polymer completely covering said biologically/physiologically active substance layer;
wherein
a water-soluble substance is contained in said polymer layer in dispersed state, said water-soluble substance being present at a concentration in the range from 1 to 50 weight-percent based on the weight of said biodegradable polymer and capable of being eluted in the living body such that pores are formed in said polymer layer with the elution of said water-soluble substance.

2. A stent as defined in Claim 1, wherein the stent main body is formed from a metallic material.

3. A stent as def ined in Claim 1, wherein the stent main body is formed from a polymeric material.

4. A stent as defined in Claim 1, wherein the biologically/physiologically active substance layer is composed solely of a biologically/physiologically active substance.

5. A stent as defined in Claim 1, wherein the biologically/physiologically active substance layer is formed from a mixture of a biologically/physiologically active substance and a low-molecular weight water-soluble material.

6. A stent as defined in Claim 1, wherein the biologically/physiologically active substance is at least onemember selected from the group consisting of carcinostatic, immunosuppressive, antibiotic, antirheumatic, antithrombotic, antihyperlipidemic, ACE inhibitor, calcium antagonist, integrin inhibitor, antiallergic, antioxidant, GPIIb/IIIa antagonist, retinoid, flavonoid, carotenoid, lipid improving agent, DNA synthesis inhibitor, tyrosine kinase inhibitor, antiplatelet, vascular smooth muscle antiproliferative agent, antiinflammatory agent, living body-derived material, interferon, and NO production accelerator.

7. A stent as defined in Claim 1, wherein the biodegradable polymer is any of polylactic acid, polyglycolic acid, polyhydroxybutyric acid, and polycaprolactone, or a mixture of two or more thereof in which components are simply mixed together or covalently bonded together.

8. A stent as defined in Claim 1, wherein the water-soluble substance is a water-soluble polymer.

9. A stent as defined in Claim 8, wherein the water-soluble polymer is at least one member selected from the group consisting of water-soluble polyalkylene glycol, polyvinylpyrrolidone, polysaccharide, polyvinyl alcohol, polyacrylamide, and polyacrylate salt.

10. A stent as defined in Claim 1, wherein the water-soluble substance is a water-soluble organic compound.

11. A stent as defined in Claim 10, wherein the water-soluble organic compound is glycerin or propylene glycol.

## Patentansprüche

1. Stent zur Implantation in einen Kanal eines lebenden Körpers, mit:
1) einem Hauptkörper des Stents;
2) einer Schicht einer biologisch/physiologisch aktiven Substanz, die aus mindestens einer Art von biologisch/physiologisch aktiver Substanz gebildet ist und auf der Oberfläche des Hauptkörpers des Stents bereitgestellt wird; und
3) einer Polymerschicht aus einem biologisch abbaubaren Polymer, die die Schicht einer biologisch/physiologisch aktiven Substanz vollständig bedeckt;
**dadurch gekennzeichnet, dass** eine wasserlösliche Substanz in dispergiertem Zustand in der Polymerschicht enthalten ist, wobei die wasserlösliche Substanz in einer Konzentration im Bereich von 1 bis 50 Gew.-% bezogen auf das Gewicht des biologisch abbaubaren Polymers vorhanden ist und in dem lebenden Körper eluiert werden kann, so dass beim Eluieren der wasserlöslichen Substanz Poren in der Polymerschicht gebildet werden.

2. Stent nach Anspruch 1, **dadurch gekennzeichnet, dass** der Hauptkörper des Stents aus einem metallischen Material gebildet ist.

3. Stent nach Anspruch 1, **dadurch gekennzeichnet, dass** der Hauptkörper des Stents aus einem polymeren Material gebildet ist.

4. Stent nach Anspruch 1, **dadurch gekennzeichnet, dass** die Schicht einer biologisch/physiologisch aktiven Substanz ausschließlich aus einer biologisch/physiologisch aktiven Substanz besteht.

5. Stent nach Anspruch 1, **dadurch gekennzeichnet, dass** die Schicht einer biologisch/physiologisch aktiven Substanz aus einer Mischung aus einer biologisch/physiologisch aktiven Substanz und einem wasserlöslichen Material von niedrigem Molekulargewicht gebildet ist.

6. Stent nach Anspruch 1, **dadurch gekennzeichnet, dass** die biologisch/physiologisch aktive Substanz mindestens ein Element aus der Gruppe der Karzinostatika, Immunsuppressiva, Antibiotika, Antirheumatika, Antithrombotika, Lipidsenker, ACE-Inhibitoren, Calciumantagonisten, Integrininhibitoren, Antiallergika, Antioxidanzien, GPIIb/IIIa-Antagonisten, Retinoide, Flavonoide, Carotinoide, lipidverbessernden Mittel, DNA-Synthese-Inhibitoren, Tyrosinkinase-Inhibitoren, Antithrombozytenmittel, Mittel zur Verhinderung einer Proliferation der glatten Gefäßmuskulatur, entzündungshemmenden Mittel, aus einem lebenden Körper gewonnenen Materialien, Interferon und Beschleuniger einer NICHT-Produktion ist.

7. Stent nach Anspruch 1, **dadurch gekennzeichnet, dass** das biologisch abbaubare Polymer jedes sein kann von Polymilchsäure, Polyglycolsäure, Polyhydroxybuttersäure und Polycaprolacton oder eine Mischung von zwei oder mehr davon, wobei die Komponenten einfach zusammengemischt oder kovalent aneinander gebunden sind.

8. Stent nach Anspruch 1, **dadurch gekennzeichnet, dass** die wasserlösliche Substanz ein wasserlösliches Polymer ist.

9. Stent nach Anspruch 8, **dadurch gekennzeichnet, dass** das wasserlösliche Polymer mindestens ein Element aus der aus wasserlöslichem Polyalkylenglycol, Polyvinylpyrrolidon, Polysaccharid, Polyvinylalkohol, Polyacrylamid und Polyacrylatsalz bestehenden Gruppe ist.

10. Stent nach Anspruch 1, **dadurch gekennzeichnet, dass** die wasserlösliche Substanz eine wasserlösliche organische Verbindung ist.

11. Stent nach Anspruch 10, **dadurch gekennzeichnet, dass** die wasserlösliche organische Verbindung Glycerin oder Propylenglycol ist.

## Revendications

1. Stent (prothèse endovasculaire) à implanter dans un conduit chez un être vivant, qui comporte :
1) un corps principal de stent,
2) une couche de substance biologiquement/physiologiquement active, constituée d'au moins un principe biologiquement/physiologiquement actif et placée à la surface dudit corps principal de stent,
3) et une couche de polymère biodégradable qui recouvre complètement
ladite couche de substance biologiquement/physiologiquement active, et dans lequel ladite couche de polymère contient, à l'état dispersé, une substance hydrosoluble, qui s'y trouve en une quantité représentant de 1 à 50 % du poids dudit polymère biodégradable et qui peut être éluée dans l'être vivant, de sorte que des pores se forment au sein de cette couche de polymère quand cette substance hydrosoluble est éluée.

2. Stent conforme à la revendication 1, dont le corps principal est fait d'un matériau métallique.

3. Stent conforme à la revendication 1, dont le corps principal est fait d'un matériau polymère.

4. Stent conforme à la revendication 1, dans lequel la couche de substance biologiquement/physiologiquement active est uniquement constituée d'une substance biologiquement/physiologiquement active.

5. Stent conforme à la revendication 1, dans lequel la couche de substance biologiquement/physiologiquement active est constituée d'un mélange d'une substance biologiquement/physiologiquement active et d'un matériau hydrosoluble à bas poids moléculaire.

6. Stent conforme à la revendication 1, dans lequel la substance biologiquement/physiologiquement active est au moins un élément de l'ensemble constitué par les cancérostatiques, immunosuppresseurs, antibiotiques, antirhumatismaux, antithrombotiques, antihyperlipidémiants, inhibiteurs de l'ACE, antagonistes du calcium, inhibiteurs d'intégrine, antiallergiques, antioxydants, antagonistes des récepteurs GPIIb/IIIa, rétinoïdes, flavonoïdes, caroténoïdes, agents améliorant le métabolisme des lipides, inhibiteurs de synthèse d'ADN, inhibiteurs de tyrosine kinase, agents antiplaquettaires, antiproliférants bloquant la prolifération des cellules des muscles lisses vasculaires, anti-inflammatoires, substances dérivées d'êtres vivants, interférons et accélérateurs de production de NO.

7. Stent conforme à la revendication 1, dans lequel le polymère biodégradable est l'un des poly(acide lactique), poly(acide glycolique), poly-(acide hydroxybutyrique) et poly(caprolactone), ou une combinaison de deux d'entre eux ou plus dans laquelle les composants sont simplement mélangés ensemble ou bien associés ensemble par liaisons covalentes.

8. Stent conforme à la revendication 1, dans lequel la substance hydrosoluble est un polymère hydrosoluble.

9. Stent conforme à la revendication 8, dans lequel le polymère hydrosoluble est au moins un élément de l'ensemble formé par les polymères hydrosolubles de type polyalkylèneglycol, poly(vinyl-pyrrolidone), polysaccharides, poly(alcool vinylique), polyacrylamide et sel polyacrylate.

10. Stent conforme à la revendication 1, dans lequel la substance hydrosoluble est un composé organique hydrosoluble.

11. Stent conforme à la revendication 10, dans lequel le composé organique hydrosoluble est du glycérol ou du propylèneglycol.
